Europäisches Patentamt

European Patent Office

Office européen des brevets

(19)

(11) Numéro de publication: **0 113 608**
**B1**

(12) **FASCICULE DE BREVET EUROPEEN**

(45) Date de publication du fascicule du brevet: **16.05.90**

(21) Numéro de dépôt: **83402303.8**

(22) Date de dépôt: **30.11.83**

(51) Int. Cl.⁵: **A 61 K 45/06,** A 61 K 35/72,
A 61 K 35/66

(54) Association permettant d'améliorer l'absorption de divers cations.

(30) Priorité: **01.12.82 FR 8220124**

(43) Date de publication de la demande:
**18.07.84 Bulletin 84/29**

(45) Mention de la délivrance du brevet:
**16.05.90 Bulletin 90/20**

(84) Etats contractants désignés:
**AT BE CH DE GB IT LI LU NL SE**

(56) Documents cités:
**US-A-3 928 567**
**US-A-4 591 503**

**DICTIONNAIRE VIDAL, 1981, O.V.P., Paris, FR
ROTE LISTE, Editio Cantor, Aulendorf/Württ.,
DE, page 772 et 1262**

**Le dossier contient des informations techniques
présentées postérieurement au dépôt de la
demande et ne figurant pas dans le présent
fascicule.**

(73) Titulaire: **Grimberg, Georges Serge**
**123 rue de l'Université**
**F-75007 Paris (FR)**

(72) Inventeur: **Grimberg, Georges Serge**
**123 rue de l'Université**
**F-75007 Paris (FR)**

(74) Mandataire: **Madeuf, René Louis et al**
**Cabinet Madeuf Conseils en Propriété**
**Industrielle 3, Avenue Bugeaud**
**F-75116 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 113 608 B1

## Description

La présente invention a pour objet une association permettant d'améliorer l'absorption des cations par l'organisme quand ces cations sont administrés par voie buccale à base de levure d'or et d'un cation de calcium ou de magnésium.

Pour cela, on associe un sel du cation à un microorganisme.

Conformément à l'invention, l'association permettant d'améliorer l'absorption des cations par l'organisme et les médicaments qui en découlent se caractérisent en ce qu'ils contiennent un sel organique et/ou minéral associé à un microorganisme.

On utilise déjà depuis de nombreuses années, en thérapeutique et par voie buccale, différents sels de calcium et même l'association de ces sels peut être présente dans une même forme.

Après avoir administré des quantités croissantes de $Ca^{++}$, la dose maximum usuelle est actuellement fixée à 500 mg de $Ca^{++}$, deux à trois fois par jour, et cela parce qu'une quantité plus importante n'amène pas une calcémie plus importante.

L'objet de la présente invention est d'augmenter la calcémie donnée par une même dose de calcium.

### Exemple N° 1

Anion—Ca quantité suffisante pour avoir 500 mg de $Ca^{++}$ Levure: 100 mg.

Excipient galénique et/ou aromatique Q.S.P. pour une dose.

On constate que le médicament, objet de la présente invention, peut être formé par un sel de calcium organique et/ou minéral, soluble ou insoluble, et qu'à la place de la levure, on peut mettre d'autres microorganismes type lactobacille ou autres.

### Toxicologie

La D L 50 est voisine de 1.000 mg de calcium éléments par kilo de sujet.

### Pharmacologie animale

100 rats au sevrage sont soumis à un régime alimentaire synthétique totalement carencé en calcium.

20 rats au sevrage sont soumis à un régime alimentaire normal et ont servi de lot témoin.

Les 100 rats carencés ont été subdivisés en 5 lots de 20 rats: 10 mâles, 10 femelles.

Le lot carencé N° 1 n'a pas reçu de $Ca^{++}$.

Le lot carencé N° 2 a reçu une préparation calcique contenant un sel organique de calcium et une quantité correspondante de levure, dénomée dans la suite du texte Médicament A.

Le lot carencé N° 3 a reçu le même sel de calcium que le lot N° 2 et la même quantité mais pas de levure dénommée dans la suite du texte, Médicament B.

Le lot carencé N° 4 a reçu une préparation calcique contenant un sel minéral de calcium et une qunatité correspondante de levure, dénomée dans la suite du texte Médicament C.

Le lot carencé N° 5 a reçu la même préparation calcique que le lot N° 4 et la même quantité mais sans levure, dénommée dans la suite du texte Médicament D.

Après soixante jours de traitement, la calcémie moyenne des rats lots 2 et 4 est statistiquement supérieure à celle des lots 3 et 5.

D'autre part, les calcémies des lots 2 et 4 sont semblables.

Donc, le médicament calcium-levure permet une nette amélioration de la thérapeutique calcique.

### Pharmacologie clinique

30 patients sont divisés en deux groupes de 15.

Aux médicaments A, B, C, D, sont ajoutés 10 microcuries de $Ca^{45}$.

Le groupe I— reçoit au temps $J_0$ le médicament A+10 microcuries de $Ca^{45}$.

reçoit au temps $J_0$+30 (trente jours après) le médicament B+10 microcuries de $Ca^{45}$.

Le groupe II—reçoit au tmeps $J_0$ le médicament D,+10 microcuries de $Ca^{45}$.

reçoit au temps $J_0$+30, le médicament C+10 microcuries de $Ca^{45}$.

Chaque patient est son propre témoin. La mesure de la radioactivité permet de montrer d'une manière statistiquement significative que les médicaments A et C sont supérieurs à B et D.

### Therapeutique

Différents malades, susceptibles de recevoir une thérapeutique calcique, ont vu leurs tonus revenir plus rapidement qu'avec les autres calcium.

### Exemple N° 2

Le même genre d'étude a été fait avec le magnésium. La pharmacologie animale démontre nettement que le sulfate de magnésium avec levure donne un magnésium mieux absorbé que le même sulfate de magnésium sans levure.

La pharmacologie humaine faite avec du magnésium 28 montre, de la même manière, le rôle du microorganisme.

### Exemple N° 3

De l'or marqué a été utilisé par voie buccale, la démonstration là encore est faite: avec microorganisme, l'absorption est meilleure.

Le cuivre, l'étain, le cobalt, le zinc, l'argent ont été testés ainsi que d'autres cations de valence quelconque.

La multiplicité de ces exemples démontre bien le rôle des microorganismes tels que la levure en association avec un cation.

Il est à noter également que le microorganisme ajouté à la préparation, qui peut être présentée sous beaucoup de formes habituelles en thérapeutique, peut être un élément vivant ou mort.

De plus, la quantité en microorganisme présentée dans la préparation médicamenteuse peut varier et être plus ou moins importante que la quantité de cations.

**Revendication**

Association medicamenteuse permettant d'améliorer l'absorption des cations par l'organisme, caractérisée en ce qu'elle est constituée par:
a) la levure
b) de l'or
c) un cation de calcium ou de magnesium.

**Patentanspruch**

Arzenieliche Verbindung die es ermöglicht, die Absorption der Kationen durch den Organismus zu verbessern, dadurch gekennzeichnet, dass diese Verbindung
a) Hefe,
b) Gold,
c) ein Kalzium- oder Magensiumkation enthält.

**Claim**

Medicamentous association enabling to improve the absorption of cations by the human system, characterized in that it is formed with:
a) yeast
b) gold
c) a cation of calcium or magnesium.